# EUROPEAN PATENT APPLICATION

(11) **EP 1 326 432 A2**
(43) Date of publication of application: **09.07.2003**
(21) Application number: 02028130.9
(22) Date of filing: 18.12.2002
(51) Int. Cl.: H04N 5/225, H04N 13/00

(54) **Device, system and method for capturing in-vivo images with three-dimensional aspects**

(30) Priority: 18.12.2001 US 340256 P
(71) Applicant: Given Imaging Ltd., 20692 Yoqneam Ilite (IL)
(72) Inventor: Glukhovsky, Arkady, 36790 Nesher (IL); Kislev, Hanoch, 30900 Zichron Yaakov (IL); Meron, Gavriel, 49556 Petach Tikva (IL)
(74) Representative: VOSSIUS & PARTNER

(57) **Abstract**

In-vivo images including three-dimensional or surface orientation information may be captured and viewed. An in-vivo site is illuminated by a plurality of sources, and the resulting reflected images may be used to provide three-dimensional or surface orientation information on the in-vivo site. The system may include a swallowable capsule.

## Description

The present invention relates to an in-vivo imaging device and a system and method such as for imaging a body lumen; more specifically, to a device and method providing stereoscopic or three-dimensional images of and determination of the surface orientation of an in-vivo site.

Various in-vivo measurement systems for examining a body lumen are known in the art. The most common type of system is an endoscope. Endoscopes are devices which include a tube (either rigid or flexible) and other equipment such as an optical system, and which are introduced into the body to view the interior. In-vivo imager systems exist which capture images using a swallowable capsule. In one such system, the imager system captures and transmits images of the GI tract to an external recording device while the capsule passes through the GI lumen.

Devices such as endoscopes, swallowable capsules and other imaging systems typically provide two dimensional images of body cavities, such as, for example, the GI tract. Thus, the surface orientation and three-dimensional nature of the site cannot be easily determined. Certain structures or conditions existing in body cavities have three-dimensional nature, the capture and presentation of which aids in their diagnosis or understanding. For example, in the GI tract, the viewing of, for example, polyps, lesions, open wounds or sores, swelling, or abnormal patterns of villi may be enhanced with three-dimensional, surface orientation or image depth information. When used herein, the surface orientation of an object or a surface is meant to include the information on the three-dimensional aspects of the object or surface, including but not limited to bumps, protrusions, raised portions, indentations, and depressions.

Certain endoscopes providing three-dimensional measurements exist, such as that described by Yokata, U.S. Patent 4,656,508. However, such systems are relatively complex and expensive, and take up enough space so that they may not be used with smaller imaging systems, such as swallowable imaging systems. Furthermore, surface feature reconstruction is more difficult with such systems.

Therefore, there is a need for an in-vivo imaging system which effectively and easily captures the three-dimensional aspects of the structures viewed.

An embodiment of the system and method of the present invention provides in-vivo images including stereoscopic, three-dimensional, surface orientation or image depth information. An in-vivo site is illuminated by a plurality of sources, and the resulting reflected images may be used to provide three-dimensional or surface orientation information on the in-vivo site. In one embodiment, the system includes a swallowable capsule.

In one embodiment, a system for imaging an in-vivo site includes a swallowable capsule including at least an imager; and a plurality of illumination sources, wherein each of the plurality of illumination sources are operated in a separate time period. At least two of the plurality of illumination sources may be configured to illuminate an in vivo site from different angles. At least one of the plurality of illumination sources may produce an illumination level which differs from the illumination level produced by a different one of the plurality of illumination sources. In one embodiment, each of the plurality of illumination sources may produce illumination of the same spectrum. The capsule may include a transmitter, and may include a battery. The capsule may include a controller configured to control the illumination sources in a selective manner. The system may include a receiving unit configured to receive transmitted image data. The system may include a processor configured to create from an image pair a single image portraying three-dimensional and surface orientation information.

Different illumination sources may produce, for example, infra red, UV, white, or other illumination.

In one embodiment, an in-vivo imaging system for imaging an in-vivo site includes a swallowable capsule including at least an imager; and a plurality of illumination sources, wherein each of the plurality of illumination sources is capable of producing a different spectrum. The capsule may include a transmitter. The capsule may include a mosaic filter. The system may include a receiving unit configured to receive transmitted image data. A processor may be configured to create from an image pair a single image portraying three-dimensional and surface orientation information.

In one embodiment, a method for capturing in-vivo images includes: illuminating an in vivo site with a set of illumination sources non-simultaneously; and capturing a set images of the site using an imager contained within a swallowable capsule, at least two images in the set illuminated using different subsets of illumination sources. The method may include transmitting the images via a wireless link. The method may include passing light through a segmented filter. The step of illuminating an in vivo site may include illuminating in at least two different illumination levels.

In one embodiment, a method for capturing in-vivo images includes: illuminating an in vivo sight with at least two illumination sources, said illumination sources producing different spectrums; and capturing a set of images of the site using an imager contained within a swallowable capsule. The method may include transmitting the images via a wireless link. In one embodiment, the spectral content of at least two of the illumination sources is the same, and method includes: when capturing a first image using a first of the illumination sources, providing illumination from a third illumination source, wherein the illumination from the third illumination source differs in its spectral content from that of a second of the illumination sources.

In one embodiment, an in-vivo imaging system for imaging an in-vivo site includes an in-vivo imaging system for imaging an in-vivo site, the system including a swallowable capsule, the capsule including: an imager; and a plurality of illumination sources, wherein the plurality of illumination sources are spaced from one another and selectively operable, such that the combination of the plurality of reflected images produced by illumination from the plurality of illumination sources provides information on the three-dimensional aspects of the in-vivo site. Each of the plurality of illumination sources may be operated in a separate time period, or alternately the same time period. At least one of the plurality of illumination sources may produce illumination in a spectrum which differs from the spectrum of illumination produced by a different one of the plurality of illumination sources. Alternately, each illumination source may produce illumination of the same spectrum.

In one embodiment, an in-vivo imaging system for imaging an in-vivo site includes an imager; a transmitter; and a plurality of illumination sources, wherein at least one of the plurality of illumination sources produces illumination in a spectrum which differs from the illumination produced by at least a second one of the plurality of illumination sources.

In one embodiment, an in-vivo imaging system for imaging an in-vivo site includes an imager; a transmitter; and a plurality of illumination sources, wherein each illumination source provides light from a different angle, each illumination source being selectively operable. In one embodiment, each of the plurality of illumination sources are operated in a separate time period.

In one embodiment, a system for presenting images includes a processor accepting a series of images from an in-vivo imager, the series of images including surface orientation information, and outputting graphics images displaying such images to a user such that the user may perceive surface orientation aspects of the images. The in-vivo imager may be contained in a capsule. For each image the surface orientation information may be recorded by at least a plurality of sub-images, each sub-image including an image of a site using a different lighting perspective.

In one embodiment, a system for presenting images includes a processor means accepting a series of images from an in-vivo imager, the series of images including surface orientation information, and outputting graphics images displaying such images to a user such that the user may perceive stereoscopic information.

In one embodiment, a method for presenting images includes: accepting a series of images from an in-vivo imager, the series of images including surface orientation information; and outputting graphics images displaying such images to a user such that the user may perceive surface orientation aspects of the images. The in-vivo imager may be contained in a capsule. For each image the surface orientation information may be recorded by at least a plurality of sub-images, each sub-image including an image of a site using a different lighting perspective.

Fig. 1 depicts an in-vivo image capture device according to one embodiment of the present invention.

Fig. 2 depicts a schematic diagram of an in-vivo imaging system according to one embodiment of the present invention.

Fig. 3 is a flow chart illustrating a method according to an embodiment of the present invention.

Fig. 4 depicts an in-vivo image capture device according to one embodiment of the present invention.

Fig. 5 depicts an in-vivo image capture device according to one embodiment of the present invention.

Fig. 6 depicts a portion of a filter used with an embodiment of the present invention.

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention.

Embodiments of US Patent No. 5,604,531, assigned to the common assignee of the present application and incorporated herein by reference, describe an in vivo camera system, which is carried by a swallowable capsule. Another in-vivo imaging system is described in International Application Publication No WO01/65995 published 13 September 2001, assigned to the common assignee of the present application and incorporated herein by reference. While embodiments of the system and method of the present invention may be used with devices and methods described in U.S. Patent 5,604,531 and/or International Application Publication No WO01/65995, embodiments of the present invention may be used with other in-vivo imaging systems, having other configurations.

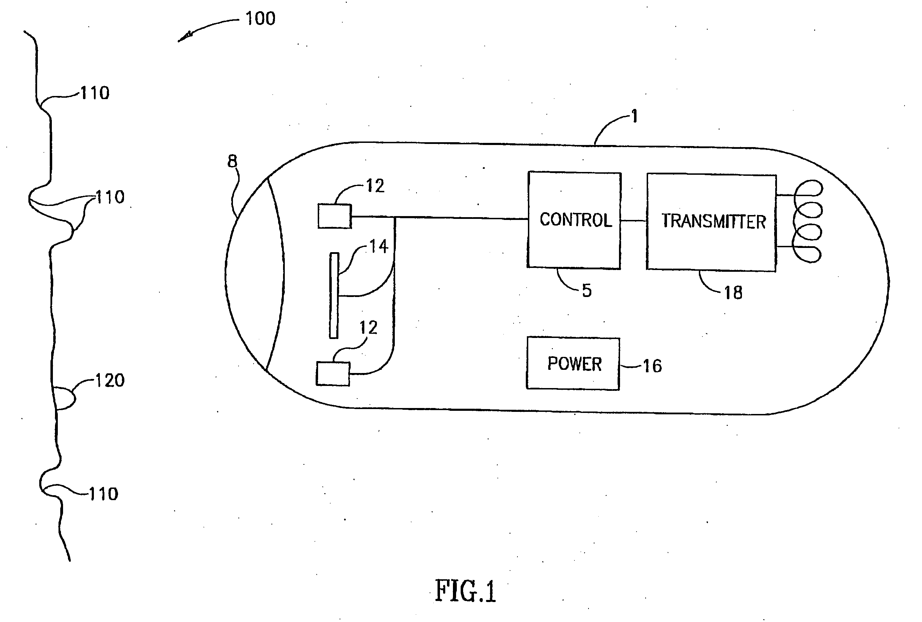

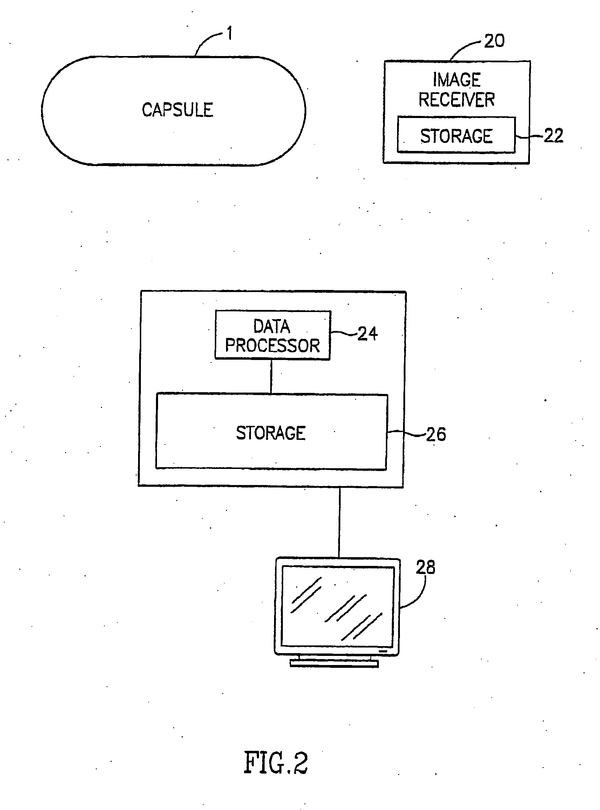

Reference is made to Fig. 1, which depicts an in-vivo image capture device according to one embodiment of the present invention. In an exemplary embodiment, the in-vivo image capture device is a capsule 1 which comprises a plurality of illumination sources 12 and 12', such as light emitting diodes (LEDs), for illuminating the body lumen, and an imager 14, such as a CMOS imager, for obtaining images of an in-vivo site 100. In an embodiment where the image capture device is a capsule 1 which moves through the GI tract, the view of the in-vivo site 100 captured changes with the movement of the image capture device. Preferably, periodically, a representation of a view of the site 100 is captured including stereoscopic, three-dimensional, surface orientation or image depth information. The illumination sources 12 and 12' and the imager 14 are preferably positioned behind an optical window 8. An optical system, including, for example, lenses or mirrors (not shown), or including optical window 8, may aid in focusing reflected electromagnetic energy onto the imager. A control unit 5 is connected to each of the illumination sources 12 and 12' and to imager 14, to synchronize the preferably nonoverlapping periodic illumination of the in-vivo site by each of illumination sources 12 and 12' with the capturing of images by imager 14. The capsule preferably includes a power source 16, such as a battery, which provides power to elements of the capsule 1, and a transmitter and antenna 18 for transmitting images obtained by imager 14 and possibly other information to a receiving device (Fig. 2) via a wireless link. The control unit 5 may be any sort of device or controller enabling the control of components. For example, a microchip, a microcontroller, or a device acting on remote commands may be used.

While in an exemplary embodiment, the illumination produced by the illumination sources 12 and 12' is substantially white light, in alternate embodiments different illumination may be produced. For example, infra-red, red, blue or green light may be produced. Furthermore, while in one embodiment illumination sources 12 and 12' produce the same spectrum of illumination, in alternate embodiments each may produce different spectra. Each of illumination sources 12 and 12' may be, for example, individual sources, such as lamps or LEDs, may be sets of sources, such as certain LEDs in a ring of LEDs, or may be overlapping sets of sources.

Preferably, the capsule 1 is swallowed by a patient and traverses the patient's GI tract. Preferably, the capsule 1 is a swallowable capsule capturing images, but may be another sort of device and may collect information in addition to image information. For example, system and method according to an embodiment of the present invention may employ a device implanted within a patient's abdomen. Furthermore, in an embodiment including a capsule different configurations of components and systems may be included the capsule. For example, the control unit may be incorporated in the transmitter, and an imager other than a CMOS imager may be used.

In an exemplary embodiment, while the capsule 1 traverses a patient's GI tract, the capsule 1 transmits image and possibly other data to components located outside the patient's body which receive and process the data. Preferably, two images using different illumination sources are captured 20 milliseconds apart, stored in the capsule 1, and transmitted as one burst of information; one second later another two images are captured. Other time differentials may be used. The two images may be transmitted as two separate images or, alternately, processed and interlaced or combined into one image before transmission. The images may be combined by interleaving by bit or by pixel before transmission, or otherwise interleaved or combined. Alternately, the images may be multiplexed through known methods. In alternate embodiments, other rates of imaging and other timing schemes may be used. Since the capsule 1 moves through the GI tract (with possibly stationary periods), typically each image frame is different; thus successive images of the in-vivo site 100 differ.

Reference is made to Fig. 2, which depicts a schematic diagram of an in-vivo imaging system according to one embodiment of the present invention. Located outside the patient's body in one or more locations are an image receiver 20, for receiving image information from an image capture device, an image receiver storage unit 22, for storing image data at the image receiver 20, a data processor 24 for processing image data, a data processor storage unit 26, for storing image data used by the data processor 24, and an image monitor 28, for displaying, *inter alia*, the images transmitted by the capsule 1 and recorded by the image receiver 20. The image receiver 20 preferably includes an antenna or antenna array 15. Preferably, the image receiver 20 and image receiver storage unit 22 are small and portable, and are worn on the patient's body during recording of the images. Preferably, the data processor 24, data processor storage unit 26 and monitor 28 are part of a personal computer or workstation which includes standard components such as processor 24, a memory, a disk drive, and input-output devices, although alternate configurations are possible. Other systems for capturing, processing and recording image and other data from the in-vivo image capture device according to embodiments of the invention may be used. For example, an in-vivo image capture device may be attached by a wire to a recording device.

In certain embodiments, the image capture device includes plurality of preferably selectively operable or switchable light sources, allowing for an inexpensive, easy and compact system for capturing the three dimensional aspects of an in-vivo site. Preferably, the light sources are selectively operable in a high speed manner. In one embodiment of the present invention, three-dimensional data (*e.g.*, image depth data) and surface orientation data of an in-vivo site is obtained by illuminating the site from a plurality of illumination sources, each at a different angle or orientation to the in-vivo site. The illumination from different angles or orientations may be achieved by spacing the illumination sources from one another by alternative methods, such as co-locating illumination sources producing illumination in different directions.

In such an embodiment, the different images produced by the illumination reflected from the same site may be combined, viewed separately, viewed together, or processed to provide to a user information on the three-dimensional aspects of the site. In one embodiment, each source is selectively operable, and illuminates the site during different time periods. The time periods may be separate, or may be overlapping. In another embodiment, the sources may provide illumination simultaneously. If illuminated by multiple sources at different times, images of the site are obtained during each of the illumination periods, each image depicting the site illuminated from each of the illumination sources at their respective angles to the site. The images obtained during each of the periodic illuminations depict different perspectives. The shadows caused by protrusions and irregularities in the surface of the site, and the shading and coloring of the surface topography, differ in each of the images. For example, the shadows vary in size and direction depending on the angle of the illumination source.

In alternate embodiments, rather than selectively operating illumination sources to be completely on or completely off, certain sources may be dimmed or have their illumination varied at certain times, thereby producing effects enabling the capture of surface orientation and three-dimensional information. Furthermore, in certain embodiment, the various illumination sources may provide different spectra of illumination (*e.g.*, red, green or blue spectra, infra-red spectra or UV spectra). In such embodiments, the illumination provided can be arranged in such way that the illumination direction is different for each channel having a different spectrum.

The images may be processed to obtain data on the surface orientation of the in-vivo site, and may be presented to a user in formats allowing for the display of three-dimensional or surface orientation data. Preferably, a system and a method according to an embodiment the present invention utilize a broad spectrum of electromagnetic energy and do not require the use of more than one image sensor, such that existing in-vivo imagining systems may be easily utilized with such an embodiment. In alternate embodiments, multiple image sensors may be used.

Preferably, for each view or site, information is gathered which includes a plurality of sub-images, each sub-image including an image of a site using a different lighting perspective. Referring to Fig. 1, in-vivo site 100 includes irregularities 110 and may include pathologies, such as polyp 120. Irregularities 110 and polyp 120 have three-dimensional characteristics. During operation, electromagnetic radiation from the illumination source 12, such as visible light rays, illuminates the in-vivo site 100 during a first period at a first angle. The imager 14 is synchronized to obtain an image of the in-vivo site during the period of illumination by illumination source 12. Preferably, the illumination sources 12 and 12' and the imager 14 are under the control of control unit 5. The image obtained by imager 14 depicts the in-vivo site 100 as illuminated from the first angle, including shadows. The image captured by imager 14 is transmitted by way of the transmitter and antenna 18 to the receiver 20. Electromagnetic radiation from the illumination source 12' illuminates the in-vivo site 100 during a second period, preferably not overlapping with the first period, at a second angle. Since the illumination sources 12' and 12 are preferably spaced from one another and separated by a certain distance the first angle is different from the second angle and the orientation of the illumination beams differs. In an exemplary embodiment, the illumination sources are 1.5 to 3 millimeters apart, in another embodiment the illumination sources are approximately 1 centimeter apart; in alternate embodiments other distances may be used. In general, the greater the distance, the more three dimensional or surface orientation information captured. When used herein, that the illumination sources are spaced from one another indicates that the sources of the illumination at the point the illumination is projected from the device are spaced from one another.

The imager 14 is synchronized to obtain an image of the in-vivo site during the second period of illumination. The image obtained by imager 14 depicts the in-vivo site 100 as illuminated from the second angle, including shadows. In one embodiment, the illumination of illumination source 12 and illumination source 12' is sequential, and occurs with a brief separation of time, in order that the view captured by imager 14 does not change significantly in between the capture of the two images. Preferably, there is a separation of approximately 10 to 20 milliseconds between the capture of the two images. In alternate embodiments, the illumination periods of illumination sources 12 and 12' may overlap.

Data representing the images captured by imager 14 are transmitted by way of the transmitter and antenna 18 to image receiver 20 using, for example, electromagnetic radio waves. For each view of an in-vivo site a set of images (where the set may include only one image) are captured and transmitted. In one embodiment the set of images includes multiple images, each based on illumination from one of multiple illumination sources, are captured and transmitted. In other embodiments, the set of images may include only one image. In one embodiment, each of illumination source 12 and 12' are individual electromagnetic radiation sources; in further embodiments, each of illumination source 12 and 12' may include multiple electromagnetic radiation sources; for example, multiple lamps. For example, each of illumination source 12 and 12' may comprise half of a ring of illumination sources. In further embodiments, more than two illumination sources may be used, and in addition more than two views per in-vivo site may be generated. In certain embodiments, illumination sources 12 and 12' may be positions close together, but may project electromagnetic energy in different angles. In alternate embodiments other devices for illumination may be used; for example, other types of lamps, fiber optic cables, or individual illumination devices capable of altering the direction of illumination.

Image receiver 20 transfers the image data to image receiver storage unit 22. After a certain period of time of data collection, the image data stored in storage unit 22 is sent to data processor 24 or data processor storage unit 26. For example, the image receiver storage unit 22 may be taken off the patient's body and connected, via a standard data link, *e.g.*, a serial or parallel interface of known construction, to the personal computer or workstation which includes the data processor 24 and data processor storage unit 26. The image data is then transferred from the image receiver storage unit 22 to the data processor storage unit 26. Data processor 24 analyzes the data and provides the analyzed data to the image monitor 28, where a health professional views, for example, the image data and possibly other information. In alternate embodiments, the image data need not be stored, but may be transferred directly to a data processor, or may be displayed immediately.

The image data collected and stored may be stored indefinitely, transferred to other locations, or manipulated or analyzed. A health professional may use the images to diagnose pathological conditions of the GI tract, and, in addition, the system may provide information about the location of these pathologies. While, using a system where the data processor storage unit 26 first collects data and then transfers data to the data processor 24, the image data is not viewed in real time, other configurations allow for real time viewing. The image monitor 28 presents the image data, preferably in the form of still and moving pictures, and in addition may present other information. In an exemplary embodiment, the various categories of information are displayed in windows. Multiple monitors may be used to display image and other data.

Preferably, the image data recorded and transmitted by the capsule 40 is digital color image data, although in alternate embodiments other image formats may be used. In an exemplary embodiment, each frame of image data includes 256 rows of 256 pixels each, each pixel including data for color and brightness, according to known methods. For example, in each pixel, color may be represented by a mosaic of four sub-pixels, each subpixel corresponding to primaries such as red, green, or blue (where one primary is represented twice). The brightness of the overall pixel is recorded by a one byte *(i.e.,* 0-255) brightness value. Preferably, images are stored sequentially in data processor storage unit 26. The stored data is comprised of one or more pixel properties, including color and brightness.

While, preferably, information gathering, storage and processing is performed by certain units, the system and method of the present invention may be practiced with alternate configurations. Furthermore, the components gathering image information need not be contained in a capsule, but may be contained in any other vehicle suitable for traversing a lumen in a human body, such as an endoscope, stent, catheter, needle, etc.

In an exemplary embodiment, the user is presented with image data allowing the user to see the three-dimensional and surface orientation aspects of the captured images. Any suitable method of presenting image pairs to obtain dimension perception may be used. For example, for each frame, the first and second images may be presented to a viewer in a time sequence such as an alternating time sequence. In this method, any difference in surface topography between the images will be perceived as a movement, giving the illusion of depth and dimension.

In alternate embodiments, the data processor 24 or another data processing unit may process the image data to create from each image pair a two-dimensional or stereoscopic image portraying the three-dimensional and surface orientation information. The data processor may, for example, subtract aspects one image from another image to highlight differences between the images; other types of processing may be performed. The user may view the resulting images as two-dimensional images, or may view the images as stereoscopic or three-dimensional images. For example, known methods may be used, such as switched glasses, polarized glasses, or colored glasses, or any other suitable manner of delivering distinct images to the left eye and right eye of a viewer. Using switched glasses, a data processor controls which lens is opaque and which is clear at different times, allowing image data from one screen to be sent to different eyes. Using polarized or colored glasses, different image data may be sent to each eye.

In some embodiments, data processor 24 may process the image using, for example, known shape from shadow methods such as that described in 3-D Stereo Using Photemetric Ratios, Lawrence B. Wolff and Elli Angelopoulou, SPIE Vol. 2065 pp. 194-209. In such embodiments, data processor 24 compares the shadows depicted in each image pair to generate data surface orientation of the in-vivo site 100. The data processor 24 may process the images according to other methods.

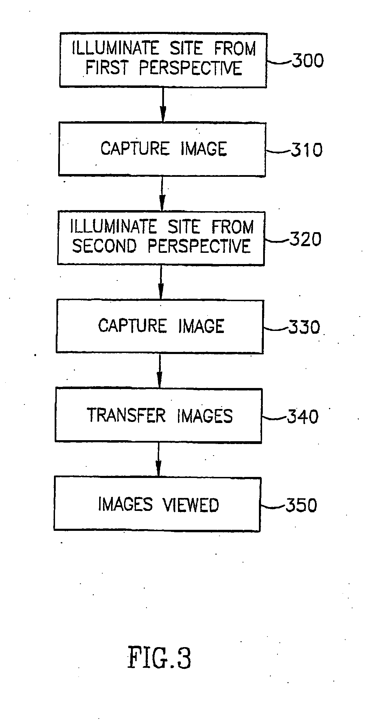

Fig. 3 is a flow chart illustrating a method according to an embodiment of the present invention.

Referring to Fig. 3, in step 300, an imaging device illuminates a site to be imaged from a first perspective. Preferably, the imaging device is a swallowable capsule; in alternate embodiments other imaging devices, such as endoscopes, may be used.

In step 310, an image is captured by the imaging device while the site is being illuminated from the first perspective.

In step 320, an imaging device illuminates a site to be imaged from a second perspective. Preferably, the illumination from the first and second perspective is provided by two illumination devices separated spatially. In alternate embodiments other methods of illumination may be used; for example, fiber optic cables, illumination devices which are co-located but which project illumination at different angles, or individual illumination devices capable of altering the direction of illumination.

In step 330, an image is captured by the imaging device while the site is being illuminated from the second perspective. In alternate embodiments more than two images may be captured for each site.

In step 340, the images are transferred from the image capture device. Preferably the images are transmitted after each set corresponding to a view of an in-vivo site are captured. In alternate embodiments, each image may be transferred after each is captured, or in other manners.

In step 350, the image data may be viewed by a user in a manner allowing the user to see the three-dimensional and surface orientation aspects of the in-vivo site.

In alternate embodiments, other steps and series of steps may be used. For example, other methods of capturing image data containing three-dimensional and surface orientation information may be used. Rather than capturing multiple images for each view of an in-vivo site, three-dimensional and surface orientation data may be included in each image obtained. In one embodiment, an image of an in-vivo site is obtained that is simultaneously illuminated by multiple illumination sources. The single image may be computationally separated into multiple images or may be displayed in a manner allowing a user to discern the three-dimensional and surface orientation data.

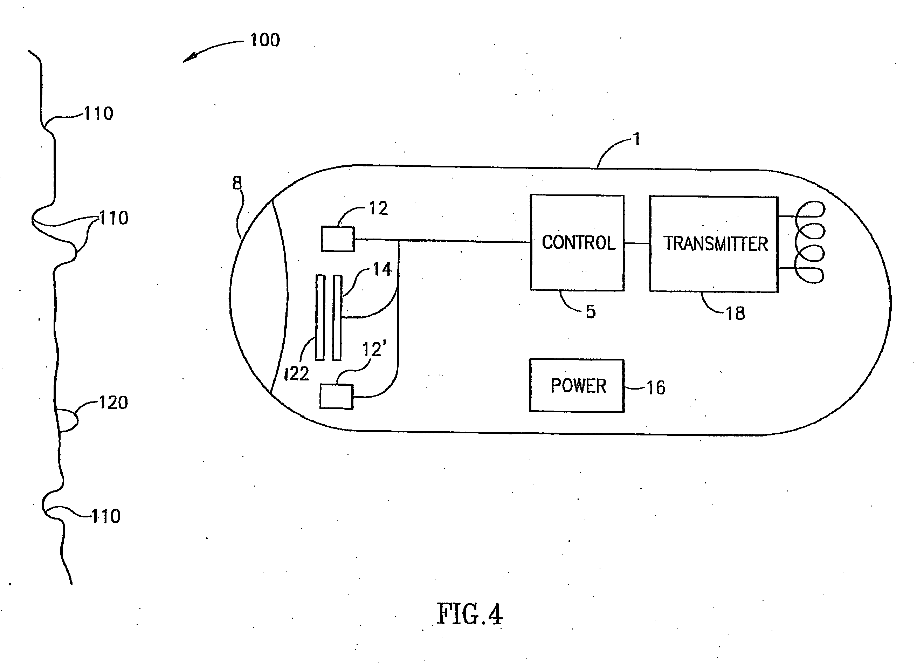

Reference is made to Fig. 4, which depicts an in-vivo image capture device according to one embodiment of the present invention. The capsule 1 functions in a similar manner to that depicted in Fig. 1, and includes a plurality of illumination sources 12 and 12', an imager 14, and an optical window 8. The capsule includes a control unit 5, a power source 16, and a transmitter and antenna 18. Each of illumination source 12 and illumination source 12' generate electromagnetic radiation of different wavelengths. The imager 14 is fitted with a filter such as a mosaic filter 122 divided into alternating segments that are sensitive to the designated bandwidths of the electromagnetic spectrum generated by the each of illumination source 12 and illumination source 12'. Each alternating segment of the mosaic filter 122 permits electromagnetic energy to reach the imager 14 only in the designated bandwidth of the electromagnetic spectrum for which it is sensitive. Each of illumination source 12 and illumination source 12' is operated simultaneously. Each image obtained by the imager 14 is composed of a plurality of segments, each segment including information from either illumination source 12 or illumination source 12'. One image containing three dimensional or surface orientation information is transmitted per view, rather than multiple images. In alternate embodiments other types of filters may be used, and the mosaic filter shown may be of a different configuration. For example, a mosaic filter with different colors or a different pattern may be used.

For example, illumination source 12 may emit red light and illumination source 12' may emit green light. In such an embodiment, the filter 22 on the imager 14 is sensitive in alternating segments to red and green light. The segments on the mosaic filter that are sensitive to red will permit red light emitted by the red illumination source during its period of illumination and reflected by the in-vivo site 100 to reach the imager. Likewise, the segments on the imager's mosaic filter that are sensitive to green will permit green light emitted by the green illumination source during its period of illumination and reflected by the in-vivo site 100 to reach the imager.

The images obtained by the imager during the respective periods of illumination may be processed (for example, by data processor 24) and displayed to the user in various manners. For example, the user may view three-dimensional images using red-green glasses. In alternate embodiments, the multiple perspective image data in the image may be used to create three-dimensional images or two-dimensional representations of three-dimensional images, such as those as described above.

In further embodiments, information on surface orientation or three-dimensional aspects may be presented to the user in other manners, for example in textual form or in graph form. For example, a graph may be created which presents the user with a depiction of the depth (positive or negative, relative to the surface of the in-vivo site 100) at various points. Such indication may be numerical, for example, a -10 to 10 scale depicting indentation or protrusion at various points, or color, with each of various colors depicting indentation or protrusion. In alternate embodiments, a view of the in-vivo site 100 may be depicted, labeled at various points with depth data (*e.g.*, numbers on a -10 to 10 scale depicting indentation or protrusion data). Further embodiments may describe the orientation of a view or various sections of a view as categories such as, for example, concave, convex, smooth or rough according to pre-defined criteria. Such data may be generated from, for example, known shape from shadow algorithms.

In a further embodiment, where each image obtained includes three-dimensional and surface orientation data, multiple illumination sources may simultaneously illuminate an in-vivo site, where certain of the illumination sources includes a marker illumination, such as infra-red or ultra-violet (UV) illumination. The spectrum of the marker illumination preferably does not overlap with the spectrum of the illumination sources. Such marker illumination may be produced by an illumination source or by an additional source. The additional marker illumination aids in distinguishing the multiple illumination sources.

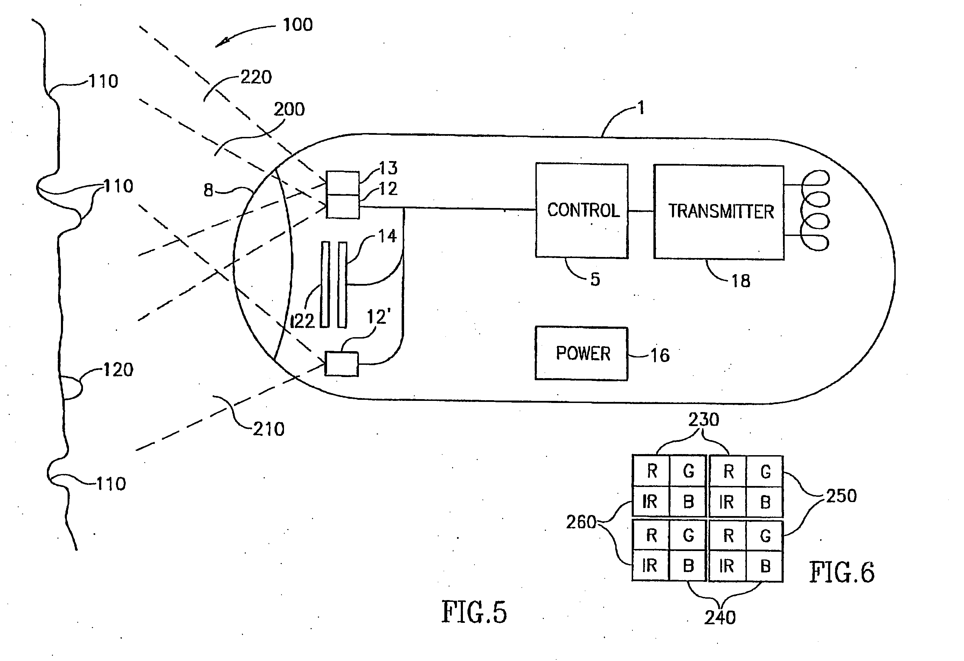

Reference is made to Fig. 5, which depicts an in-vivo image capture device according to one embodiment of the present invention. The capsule 1 functions in a similar manner to that depicted in Fig. 1, and includes a plurality of illumination sources 12 and 12', an imager 14, and an optical window 8. The capsule includes a control unit 5, a power source 16, and a transmitter and antenna 18. Preferably, each of illumination source 12 and illumination source 12' generate electromagnetic radiation of the same wavelength. Capsule 1 includes additional source 13, providing marker illumination from a position and angle substantially similar to that of illumination source 12; in effect additional source 13 adds marker illumination to illumination source 12. Rays 200 represent electromagnetic radiation produced by illumination source 12, rays 210 represent electromagnetic radiation produced by illumination source 12', and rays 220 represent electromagnetic radiation produced by source 13. Preferably, rays 220 are projected onto the in-vivo site 100 at substantially the same angle and from substantially the same position as rays 200. In one embodiment, illumination sources 12, 12' and 13 are operated simultaneously and one image is captured and transmitted. The image may be separated into different views, providing three dimensional and surface orientation information.

The imager 14 is fitted with a filter such as a mosaic filter 122 divided into alternating segments that are sensitive to different bandwidths of the electromagnetic spectrum. Certain segments allow the passage of electromagnetic radiation generated by source 13. Other segments allow the passage of electromagnetic radiation generated by illumination sources 12 and 12'. In certain embodiments segments may filter the illumination generated by sources 12 and 12' into different spectral bands, such as the red, green and blue spectra; in other embodiments segments may allow substantially the entire spectrum generated by sources 12 and 12' to pass. Each alternating segment of the mosaic filter 122 permits electromagnetic energy to reach the imager 14 only in the designated bandwidth of the electromagnetic spectrum for which it is sensitive. Preferably, each of illumination source 12 and illumination source 12' is operated simultaneously. Each image obtained by the imager 14 is composed of a plurality of segments, each segment including information from either illumination source 12 and source 12' (or a portion thereof) or source 13. In one embodiment, source 13 produces electromagnetic radiation of a certain frequency which is used to mark a perspective, such as infra-red radiation, and illumination sources 12 and 12' produce other illumination, such as visible light. In alternate embodiments, the illumination sources may produce different spectra, and thus a separate marker source may not be needed. The marker illumination may include spectra other than infra-red radiation, for example UV radiation.

Reference is made to Fig. 6, which depicts a portion of a filter used with an embodiment of the present invention. In one embodiment, the filter 22 includes a repetitive pattern of sections, each section including a plurality of cells. Each cell allows a certain spectrum of electromagnetic radiation to pass to the imager 14. For example, cells 230 allow red light to pass, cells 240 allow blue light to pass, cells 250 allow green light to pass, and cells 260 allow infra-red radiation to pass. Preferably, the filter 22 includes many sections and cells; in one embodiment one section is included for each pixel recorded by the imager 14.

After capture, the images obtained may be displayed to the user in various manners, for example using the methods described above. In one embodiment, electromagnetic energy from one section, including all cells of the section, is recorded by each pixel of the imager 14. During the processing of the image, the known frequency of the source 13 is used along with the information provided by cells 260 to produce different pixel representations for each of the two views desired. For example, the intensity of the source 13 for each pixel may be used as a marker for percentage of the electromagnetic energy for that pixel which is produced by illumination source 12.

In an alternate embodiment, an additional source need not be used to produce marker such as infra-red radiation. For example, each of two illumination sources may produce different spectra of electromagnetic radiation; the differences in the reflected and captured images may be used to provide three-dimensional information.

In a further embodiment, electromagnetic energy from each cell is recorded by one pixel of the imager 14. During the processing of the image, the known frequency of the illumination source 13 is used along with the information provided by cells 260 to produce different pixel representations for each of the two views desired. For example, the intensity of the source 13 for each pixel may be used as a marker for percentage of the electromagnetic energy for certain associated pixels gathering light in the frequency of the source (*e.g.*, source 12) associated with source 13.

It will be appreciated by persons skilled in the art that the present invention is not limited to what has been particularly shown and described hereinabove. Alternate embodiments are contemplated which fall within the scope of the invention.

## Claims

1. An in-vivo imaging system for imaging an in-vivo site, the system comprising a swallowable capsule including at least:
an imager; and
a plurality of illumination sources, wherein each of the plurality of illumination sources are operated in a separate time period.

2. The system according to claim 1 wherein at least two of the plurality of illumination sources are configured to illuminate an in vivo site from different angles.

3. The system of claim 1, wherein at least one of the plurality of illumination sources produces an illumination level which differs from the illumination level produced by a different one of the plurality of illumination sources.

4. The system of any one of the preceding claims, wherein each of the plurality of illumination sources produces illumination of the same spectrum.

5. The system of any one of the preceding claims, wherein the capsule comprises a transmitter for transmitting image data.

6. The system of any one of the preceding claims, wherein the capsule comprises a battery.

7. The system of any one of the preceding claims comprising a controller configured to control the illumination sources in a selective manner.

8. The system of any one of the preceding claims comprising a receiving unit configured to receive transmitted image data.

9. The system of any one of the preceding claims comprising a processor configured to create from an image pair a single image portraying three-dimensional and surface orientation information.

10. An in-vivo imaging system for imaging an in-vivo site, the system comprising a swallowable capsule including at least:
an imager; and
a plurality of illumination sources, wherein each of the plurality of illumination sources is capable of producing a different spectrum.

11. The system of claim 10, wherein at least one of the illumination sources produces illumination in the infra-red spectrum.

12. The system of claim 10, wherein at least one of the illumination sources produces illumination in the UV spectrum.

13. The system of any one of claims 10-12, wherein the capsule comprises a transmitter.

14. The system according to any one of claims 10-13, wherein the capsule comprises a mosaic filter.

15. The system of any one of claims 10-14 comprising a receiving unit configured to receive transmitted image data.

16. The system of claim 15 comprising a processor configured to create from an image pair a single image portraying three-dimensional and surface orientation information.

17. A method for capturing in-vivo images, the method comprising:
illuminating an in vivo site with a set of illumination sources non-simultaneously; and
capturing a set images of the site using an imager contained within a swallowable capsule, at least two images in the set illuminated using different subsets of illumination sources.

18. The method of claim 17 comprising transmitting the images via a wireless link.

19. The method of claim 17 or 18 comprising passing light trough a segmented filter.

20. The method of any one of claims 17-19, wherein the step of illuminating an in vivo site comprises illuminating in at least two different illumination levels.

21. A method for capturing in-vivo images, the method comprising:
illuminating an in vivo sight with at least two illumination sources, said illumination sources producing different spectrums; and
capturing a set of images of the site using an imager contained within a swallowable capsule.

22. The method of claim 21 comprising transmitting the images via a wireless link.

23. The method of claim 21 or 22, wherein at least one of the illumination sources produces illumination in the infra-red spectrum.

24. The method of claim 21 or 22, wherein at least one of the illumination sources produces illumination in the UV spectrum.

25. The method of claim 21 or 22, wherein at least one of the illumination sources produces substantially white light.

26. The method of claim 21, wherein the spectral content of at least two of the illumination sources is the same, the method comprising:
when capturing a first image using a first of the illumination sources, providing illumination from a third illumination source, wherein the illumination from the third illumination source differs in its spectral content from that of a second of the illumination sources.

27. An in-vivo imaging system for imaging an in-vivo site, the system comprising a swallowable capsule, said capsule comprising:
an imager; and
a plurality of illumination sources, wherein the plurality of illumination sources are spaced from one another and selectively operable, such that the combination of the plurality of reflected images produced by illumination from the plurality of illumination sources provides information on the three-dimensional aspects of the in-vivo site.

28. The system of claim 27, wherein each of the plurality of illumination sources are operated in a separate time period.

29. The system of claim 27, wherein:
each of the plurality of illumination sources are operated in the same time period; and
at least one of the plurality of illumination sources produces illumination in a spectrum which differs from the spectrum of illumination produced by a different one of the plurality of illumination sources.

30. The system of any one of claims 27-29, wherein each of the plurality of illumination sources produces illumination of the same spectrum.

31. The system of any one of claims 27-30, wherein the capsule comprises a transmitter.

32. The system of any one of claims 27-31, wherein at least one of the illumination sources produces illumination in the infra-red spectrum.

33. The system of any one of claims 27-31, wherein at least one of the illumination sources produces substantially white light illumination.

34. An in-vivo imaging system for imaging an in-vivo site, the system comprising:
an imager;
a transmitter; and
a plurality of illumination sources, wherein at least one of the plurality of illumination sources produces illumination in a spectrum which differs from the illumination produced by at least a second one of the plurality of illumination sources.

35. An in-vivo imaging system for imaging an in-vivo site, the system comprising:
an imager;
a transmitter; and
a plurality of illumination sources, wherein each illumination source provides light from a different angle, each illumination source being selectively operable.

36. The system of claim 35, wherein each of the plurality of illumination sources are operated in a separate time period.

37. A system for presenting images comprising:
a processor accepting a series of images from an in-vivo imager, the series of images including surface orientation information, and outputting graphics images displaying such images to a user such that the user may perceive surface orientation aspects of the images.

38. The system of claim 37, wherein the in-vivo imager is contained in a capsule.

39. The system of claim 37 or 38, wherein for each image the surface orientation information is recorded by at least a plurality of sub-images, each sub-image including an image of a site using a different lighting perspective.

40. A system for presenting images comprising:
a processor means accepting a series of images from an in-vivo imager, the series of images including surface orientation information, and outputting graphics images displaying such images to a user such that the user may perceive stereoscopic information.

41. A method for presenting images comprising:
accepting a series of images from an in-vivo imager, the series of images including surface orientation information; and
outputting graphics images displaying such images to a user such that the user may perceive surface orientation aspects of the images.

42. The method of claim 41, wherein the in-vivo imager is contained in a capsule.

43. The method of claim 41 or 42, wherein for each image the surface orientation information is recorded by at least a plurality of sub-images, each sub-image including an image of a site using a different lighting perspective.
